# EUROPÄISCHE PATENTSCHRIFT

(11) **EP 3 592 328 B1**
(45) Veröffentlichungstag und Bekanntmachung des Hinweises auf die Patenterteilung: **14.12.2022**
(21) Anmeldenummer: 18714444.9
(22) Anmeldetag: 28.02.2018
(51) Int. Cl.: A61K 8/34, A61K 8/73, A61Q 19/00, A61K 8/19

(54) **VERWENDUNG VON CALCIUMSALZEN IN KOSMETISCHEN ZUBEREITUNGEN**
USE OF CALCIUM SALTS IN COSMETIC PREPARATIONS
UTILISATION DE SELS DE CALCIUM DANS DES PRÉPARATIONS COSMÉTIQUES

(30) Priorität: 08.03.2017 DE 102017203797
(43) Veröffentlichungstag der Anmeldung: 15.01.2020
(73) Patentinhaber: Beiersdorf AG, 20253 Hamburg (DE)
(72) Erfinder: ALANYA-ROUSSEAU, Esma, 22769 Hamburg (DE); SKUBSCH, Kerstin, 25497 Prisdorf (DE); MÜLLER, Claudia, 25499 Tangstedt (DE); ECKERT, Julia, 22393 Hamburg (DE)
(74) Vertreter: Beiersdorf AG
(86) Internationale Anmeldenummer: PCT/EP2018/054905
(87) Internationale Veröffentlichungsnummer: WO 2018/162293

(56) Entgegenhaltungen:
- DATABASE GNPD [Online] MINTEL; 1. Oktober 2016 (2016-10-01), "Metaboliser Ultimate 2", XP002781843, Database accession no. 4301029
- DATABASE GNPD [Online] MINTEL; 1. Dezember 2014 (2014-12-01), "Cream Foundation EX", XP002781844, Database accession no. 2854101
- DATABASE GNPD [Online] MINTEL; 1. Oktober 2008 (2008-10-01), "Shimmering Body Milk", XP002781845, Database accession no. 988857
- SYLVIE VERDIER-SEVRAIN MD ET AL: "SKIN HYDRATION: A REVIEW ON ITS MOLECULAR MECHANISMS", JOURNAL OF COSMETIC DERMATOLOGY, BLACKWELL SCIENCE, OXFORD, GB, Bd. 6, Nr. 2, 1. Januar 2007 (2007-01-01), Seiten 75-82, XP001536242, ISSN: 1473-2130, DOI: 10.1111/J.1473-2165.2007.00300.X
- ROUSSEL LAURÈNE ET AL: "Glycerol as a Skin Barrier Influencing Humectant", TREATMENT OF DRY SKIN SYNDROM, SPRINGER, DE, PAGE(S) 473 - 480 , 1. Januar 2012 (2012-01-01), XP009505854, ISBN: 978-3-642-27605-7 Gefunden im Internet: URL:http://link.springer.com/10.1007/978-3 -642-27606-4_32 [gefunden am 2012-03-14]
- DATABASE GNPD [Online] MINTEL; 24 February 2013 (2013-02-24), anonymous: "Ultra Nourishing Moisturising Cream", XP055741880, retrieved from www.gnpd.com Database accession no. 2008114

## Beschreibung

Die vorliegende Erfindung betrifft die Verwendung von Calciumsalzen in Glycerin enthaltenden kosmetischen Zubereitungen zu Erhöhung der Hautbefeuchtungsleistung der Zubereitung

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte enthalten zur Hautbefeuchtung in der Regel Glycerin. Nachteilig am Stande der Technik ist jedoch der Umstand, dass Glycerin in höheren Konzentrationen dazu führt, dass die Zubereitung klebrig wird. Höhere Konzentrationen an Glycerin sind jedoch für eine effektive Hautbefeuchtung notwendig, da nur ein Teil dieser Verbindung aus der Zubereitung für die Haut befeuchtende Wirkung tatsächlich freigesetzt wird und zu diesem Zwecke zur Verfügung steht.

Es war daher die Aufgabe der vorliegenden Erfindung Wege zu finden, die Freisetzung von Glycerin aus kosmetischen Zubereitungen zu erhöhen um eine höhere Hautbefeuchtungsleistung kosmetischer Zubereitungen zu erzielen, ohne dass die Zubereitung auf der Haut klebrig wirkt.

Gelöst wird die Aufgabe durch eine Zubereitung gemäß Anspruch 1.

Zwar kennt der Fachmann grundsätzlich kosmetische Zubereitungen mit Calciumsalzen und Glycerin, doch ist bisher schlicht unbeachtet geblieben, dass sich die Freisetzung (und damit Bioverfügbarkeit) von Glycerin aus den Zubereitungen durch den Einsatz von Calciumsalzen deutlich steigern lässt. Durch die verbesserte Freisetzung von Glycerin aus der Zubereitung wird es damit möglich, geringere Mengen an Glycerin in den Zubereitungen einzuarbeiten und damit deren Klebrigkeit zu reduzieren, ohne dass die Haut befeuchtende Leistung der Zubereitung an sich geringer wird.

Ferner kennt der Stand der Technik die in der GNPD-Datenbank "Mintel" offenbarten Einträge mit den Datenbanknummern 4301029 (Metaboliser Ulitmate 2), 2854101 (Cream Foundation EX), 988857 (Shimmering Body Milk) und 2008114 (Ultra Nourishing Moiturising Cream), die ebenfalls nicht den Weg zur Erfindung weisen konnten.

Es ist erfindungsgemäß, wenn als Calciumsalz Calciumchlorid und/oder Calciumcarbonat (z.B. in Form von Heilerde) eingesetzt wird.

Es ist erfindungsgemäß besonders bevorzugt, wenn als Calciumsalz Calciumchlorid eingesetzt wird.

Es ist erfindungsgemäß, wenn die Zubereitung Calciumsalze in einer Einsatzkonzentration von 0,01 bis 3 Gewichts-%, bezogen auf das Gesamtgewicht der Zubereitung enthält. Wird Calciumchlorid oder Calciumcarbonat als Calciumsalz eingesetzt, beziehen sich diese Konzentrationsangaben auf das Calciumchlorid bzw. Calciumcarbonat.

Mit dieser Einsatzkonzentration liegt der Calciumgehalt deutlich über dem Salzgehalt des normalerweise in kosmetischen Zubereitungen eingesetzten vollentsalzten (VE-) Wassers. Der Calciumgehalt liegt auch über dem Gehalt im Trinkwasser (Beispielsweise dem des Trinkwassers des Grundwasserwerkes Borstelbek von Hamburg Wasser, das einen Gehalt von 0,005 Gew.-% Calcium aufweist (Mittelwert für das Jahr 2015)).

Vorteilhafte Ausführungsformen der vorliegenden Erfindung sind dadurch gekennzeichnet, dass die Zubereitung Glycerin in einer Konzentration von 1 bis 30 Gewichts-%, bezogen auf das Gesamtgewicht der Zubereitung, enthält. Dabei beträgt die erfindungsgemäß bevorzugte Konzentration an Glycerin in der Zubereitung von 3 bis 20 Gewichts-%, bezogen auf das Gesamtgewicht der Zubereitung.

Calciumstärkeoctenylsuccinat (INCI Calcium starch Octenylsuccinate) wird erfindungsgemäß vorteilhaft in einer Konzentration von 0,5 bis 10 Gewichts-%, bezogen auf das Gesamtgewicht der Zubereitung eingesetzt.

Es ist erfindungsgemäß von Vorteil, wenn die Zubereitung in Form einer Emulsion vorliegt. Dabei ist es erfindungsgemäß bevorzugt, wenn die Zubereitung in Form einer O/W-Emulsion vorliegt.

In einem solchen Fall sind die erfindungsgemäß vorteilhaften Ausführungsformen der Erfindung dadurch gekennzeichnet, dass die Zubereitung einen oder mehrere Emulgatoren gewählt aus der Gruppe der Verbindungen Polyglyceryl-10 stearat, Glycerylstearatcitrat, Glycerylstearat, Glycerylstearat (selbstemulgierend), Polyglyceryl-3-methylglycosedistearat, Natriumcetearylsulfat, Kaliumcetylphosphat, Natriumstearoylglutamat, Triceteareth-4-phosphat, Stearinsäure, Stearatsalze, Cetearylsulfosuccinate enthält.

Erfindungsgemäß besonders bevorzugt ist dabei eine Emulgatorkombination aus Glycerylstearat (selbstemulgierend) und Natriumcetearylsulfat

Die erfindungsgemäß vorteilhafte Einsatzkonzentration (Gesamtmenge) an Emulgatoren beträgt von 0,1 bis 5 Gewichts-%, bezogen auf das Gesamtgewicht der Zubereitung.

In diesem Zusammenhang ist auch zu erwähnen, dass es erfindungsgemäß bevorzugt ist, wenn die Zubereitung frei ist von Polyethylenglycol, Polyethylenglycolethern und Polyethylenglycolestern (sogenannten PEG-Derivaten).

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol.

Erfindungsgemäß vorteilhaft enthält die erfindungsgemäße Zubereitung elektrolytstabile Verdicker wie Acrylates/C10-30 Alkyl Acrylate Crosspolymer.

Auch die Lipidphase kann die üblichen kosmetischen Fette, Öle und Wachse enthalten. Vorteilhaft sind beispielsweise Isopropylpalmitat, Isopropylmyristat, Isopropylstearat, Isodecylneopentanoat, Octylsalicylat, Homosalat, C12-15 Alkylbenzoat, Capryl/Caprinsauretriglycerid, Di-C12-13 Alkyllactat, Cetyloleat, Butylenglycol Caprylat/Caprat, Dibutyladipat, Diisopropyladipat, Phenetylbenzoat, hydrierte Cocosglyceride, hydriertes Rizinusöl, Cetearylisononanoat.Sheabutter, Bienenwachs, Candellilawachs, Carnaubawachs, Squalan, Mandelöl, Arganöl, Jojobaöl, Macadamiaöl, Olus Oil, Decyl Oleate, Shea Butter, Kakaobutter.

Auch der Einsatz von Cera Microcristallina und Paraffinum Liquidum als Lipidkomponenten ist erfindungsgemäß vorteilhaft, Ebenso kann die erfindungsgemäße Zubereitung Dimethicon oder Cyclomethicon enthalten.

Erfindungsgemäß bevorzugt ist es, wenn die Zubereitung Myristylalkohol, Stearylalkohol und/oder Cetylalkohol enthält.

Erfindungsgemäß vorteilhafte Ausführungsformen sind auch dadurch gekennzeichnet, dass die Zubereitung einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucosid, Kreatin, Kreatinin, Taurin, ß-Alanin und/oder Licochalcon A, Panthenol, Tocopherol, Tocopherolacetat, Vitamin C ,Vitamin C Derivate, Glycyrrhiza Inflata Root Extract, Magnolienextrakt, Aloe Vera, Urea, Arctium Lappa Fruit Extract enthält.

Ferner ist es erfindungsgemäß vorteilhaft, wenn die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält.

Dabei ist der Einsatz von Ethylhexylglycerin erfindungsgemäß bevorzugt.

Ethylhexylglycerin wird erfindungsgemäß vorteilhaft in einer Konzentration von 0,1 bis 0,6 Gewichts-%, bezogen auf das Gesamtgewicht der Zubereitung eingesetzt.

Erfindungsgemäß von Vorteil ist auch der Gehalt an Phenoxyethanol in der Zubereitung.

Phenoxyethanol wird erfindungsgemäß vorteilhaft in einer Konzentration von 0,1 bis 1 Gewichts-%, bezogen auf das Gesamtgewicht der Zubereitung eingesetzt.

Erfindungsgemäß bevorzugt ist insbesondere der Einsatz einer Kombination aus Phenoxyethanol und Ethylhexylglycerin.

Erfindungsgemäß vorteilhafte Ausführungsformen der vorliegenden Erfindung sind dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydiben-zoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Titandioxid; Zinkoxid.

Erfindungsgemäß bevorzugt sind dabei jedoch Zubereitungen, die frei sind von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen und 3-lodpropargyl-*N*-butylcarbamat (IPBC).

Erfindungsgemäß vorteilhaft kann die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Ethylenbrassylat, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat, Vanillin enthalten.

### Vergleichsversuch

Es wurden die folgenden Rezepturen hergestellt:

| inci | **Rezeptur 1** | **Rezeptur 2** | **Rezeptur 3** |
|---|---|---|---|
| Panthenol + Aqua | 1,3 | 1,3 | 1,3 |
| Ethylhexylglycerin | 0,1 | 0,1 | 0,1 |
| Cera Microcristallina + Paraffinum Liquidum | 1 | 1 | 1 |
| C15-19 Alkane | 4 | 4 | 4 |
| Isopropyl Palmitate | 3 | 3 | 3 |
| Hydrogenated Coco-Glycerides | 0,5 | 0,5 | 0,5 |
| Dimethicone | 2 | 2 | 2 |
| Glyceryl Stearate SE | 1,5 | 1,5 | 1,5 |
| Glyceryl Stearate | 1 | 1 | 1 |
| Sodium Cetearyl Sulfate | 0,15 | 0,15 | 0,15 |
| Calcium Starch Octenylsuccinate + Aqua | 1 | 1 | 1 |
| Parfum | 0,35 | 0,35 | 0,35 |
| Glycerin + Aqua | 10 | 10 | 10 |
| Aqua + Sodium Hydroxide | 0,23 | 0,23 | 0,23 |
| Phenoxyethanol | 0,8 | 0,8 | 0,8 |
| Myristyl Alcohol | 1 | 1 | 1 |
| Cetearyl Alcohol | 2,5 | 2,5 | 2,5 |
| Carbomer | 0,3 | 0,3 | 0,3 |
| Calcium Chloride | 0,0 | 1,0 | 2,0 |
| Aqua | 69,27 | 68,27 | 67,27 |

Anschließend wurde die Freisetzung (Wiederfindungsrate im Rezeptorfluid) von Glycerin aus den Zubereitungen bestimmt. Hierzu wurde ein in-vitro Freisetzungs-Modell (SEM) verwendet, bei dem bei Raumtemperatur die Zubereitungen auf eine 0,025 µm dicke Nitrocellulosemembran als Barriere aufgetragen wurde (je dreimal für jede Zubereitung). Als Rezeptorfluid diente Wasser. Es wurde die Wiederfindungsrate von Glycerin im Rezepturfluid mittels HPLC-IR bestimmt.

### Wiederfindung im Rezeptur-Fluid

| | Aufgetragen e Probenmeng e in mg/cm² | Wiederfindung in der Rezeptorflüssigk eit nach 1h in % | Wiederfindung in der Rezeptorflüssigk eit nach 2,25h in % | Wiederfindung in der Rezeptorflüssigk eit nach 4h in % | Wiederfindung in der Rezeptorflüssigk eit nach 6,25h in % |
|---|---|---|---|---|---|
| Rezept ur 1 | 18,18 | 18,7 | 36,5 | 51,8 | 64,2 |
| Rezept ur 2 | 18,17 | 27,4 | 43,3 | 55,7 | 68,7 |
| Rezept ur 3 | 18,55 | 30,8 | 48,7 | 62,7 | 73,2 |

**Fazit:** Durch den Zusatz von Calciumchlorid wird signifikant mehr und schneller Glycerin aus der Zubereitung freigesetzt.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **A** | **B** | **C** | **D** |
|---|---|---|---|---|
| **Zusammensetzung in %** | **m [%]** | **m [%]** | **m [%]** | **m [%]** |
| Glyceryl Glucoside** | 0,8 | 2 | | |
| Maris Sal | 1 | | | |
| Carnitine | | | 1 | |
| Creatine | | | 2 | |
| Creatinine | | | 0,1 | |
| Coenzym Q10 | | | 0,5 | |
| Ethylhexylglycerin | | 0,5 | 0,25 | |
| Arctium Lappa Fruit Extract*** | | | | 0,2 |
| Tocopherol | | | | 0,1 |
| Dicaprylyl Ether | 3,5 | | | |
| Dimethicone | 0,5 | | | |
| Isopropyl Palmitate | 2 | 9 | | |
| Cera Microcristallina | | 3 | | |
| Paraffinum Liquidum | | 3 | | |
| Isohexadecane | | 2 | | |
| Almond Oil | | 5 | | |
| Shea Butter | | | 3 | |
| C12-15 Alkyl Benzoate | | | 1 | |
| Caprylic/Capric Triglyceride | | | | 2 |
| Hydrogenated Vegetable Oil | | | | 3 |
| Octyldodecanol | | | | 2 |
| Argan Oil | | | | 2 |
| Glyceryl Stearate SE | 2 | | | |
| Sodium Cetearyl Sulfate | 0,3 | | | |
| Stearinsäure* | | 2,5 | | |
| Natriumstearoylglutamat | | | 0,5 | |
| Glyceryl Stearate | | | 2 | 1 |
| Glyceryl Stearate Citrate | | | | 1,5 |
| Tapioca Starch | | | 1 | |
| Glycerin | 5 | 20 | 10 | 15 |
| Acrylates/C10-30 Alkyl Acrylate Crosspolymer | | 0,3 | | 0,2 |
| Aloe Vera | | 4 | | |
| Panthenol**** | | | | 1 |
| Phenoxyethanol | 0,7 | 0,4 | 0,5 | 0,4 |
| Urea | | | | 5 |
| Cetearyl Alcohol | 1 | | | |
| Carbomer | 0,2 | | 0,5 | 0,1 |
| Xanthan Gum | | 0,1 | | 0,2 |
| Alcohol Denat | 2 | | | |
| Calcium Chloride | 0,5 | 3 | 0,1 | 1 |
| Natronlauge***** q.s. auf pH Wert | pH 7,0 | pH 6,5 | pH 6,5 | pH 5,5 |
| Wasser | ad 100 | ad 100 | ad 100 | ad 100 |

| | | | | |
|---|---|---|---|---|
| * Mischung Palmitic Acid + Stearic Acid + Myristic Acid + Arachidic Acid + Oleic Acid ** 43% Glyceryl Glucoside, 54% Glycerine, 3% Wasser *** 50% Arctium Lappa Fruit Extract, 50% Glycerine **** 77% Panthenol, 23% Wasser ***** 45% Natriumhydroxid, 55% Wasser | | | | |

## Patentansprüche

1. Kosmetische Zubereitung enthaltend
a) Calciumchlorid und/oder Calciumcarbonat in einer Gesamtkonzentration von 0,01 bis 3 Gewichts-%, bezogen auf das Gesamtgewicht der Zubereitung,
b) Glycerin,
c) Calciumstärkeoctenylsuccinat (INCI Calcium starch Octenylsuccinate).

2. Zubereitung nach Anspruch 1, **dadurch gekennzeichnet, dass** als Calciumsalz Calciumchlorid eingesetzt wird.

3. Zubereitung nach einem der vorhergehenden Ansprüche, **dadurch gekennzeichnet, dass** die Zubereitung Glycerin in einer Konzentration von 1 bis 30 Gewichts-%, bezogen auf das Gesamtgewicht der Zubereitung, enthält.

4. Zubereitung nach einem der vorhergehenden Ansprüche, **dadurch gekennzeichnet, dass** die Zubereitung in Form einer Emulsion vorliegt.

5. Zubereitung nach einem der vorhergehenden Ansprüche, **dadurch gekennzeichnet, dass** die Zubereitung in Form einer O/W-Emulsion vorliegt.

6. Zubereitung nach einem der vorhergehenden Ansprüche, **dadurch gekennzeichnet, dass** die Zubereitung einen oder mehrere Emulgatoren gewählt aus der Gruppe der Verbindungen Polyglyceryl-10 stearat, Glycerylstearatcitrat, Glycerylstearat, Glycerylstearat (selbstemulgierend), Polyglyceryl-3-methylglycosedistearat, Natriumcetearylsulfat, Kaliumcetylphosphat, Natriumstearoylglutamat, Triceteareth-4-phosphat, Stearinsäure, Stearatsalze, Cetearylsulfosuccinate enthält.

7. Zubereitung nach einem der vorhergehenden Ansprüche, **dadurch gekennzeichnet, dass** die Zubereitung einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucosid, Kreatin, Kreatinin, Taurin, ß-Alanin und/oder Licochalcon A, Panthenol, Tocopherol, Tocopherolacetat, Vitamin C ,Vitamin C Derivate, Glycyrrhiza Inflata Root Extract, Magnolienextrakt, Aloe Vera, Urea, Arctium Lappa Fruit Extract enthält.

8. Zubereitung nach einem der vorhergehenden Ansprüche, **dadurch gekennzeichnet, dass** die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält.

9. Zubereitung nach einem der vorhergehenden Ansprüche, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Titandioxid; Zinkoxid.

10. Zubereitung nach einem der vorhergehenden Ansprüche, **dadurch gekennzeichnet, dass** die Zubereitung frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen und 3-Iodpropargyl-*N*-butylcarbamat (IPBC).

11. Zubereitung nach einem der vorhergehenden Ansprüche, **dadurch gekennzeichnet, dass** die Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Ethylenbrassylat, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat, Vanillin enthält.

12. Zubereitung nach einem der vorhergehenden Ansprüche, **dadurch gekennzeichnet, dass** die Zubereitung frei ist von Polyethylenglycol, Polyethylenglycolethern und Polyethylenglycolestern (sogenannten PEG-Derivaten).

13. Zubereitung nach einem der vorhergehenden Ansprüche, **dadurch gekennzeichnet, dass** die Zubereitung Myristylalkohol, Stearylalkohol und/oder Cetylalkohol enthält.

14. Zubereitung nach einem der vorhergehenden Ansprüche, **dadurch gekennzeichnet, dass** die Zubereitung Phenoxyethanol enthält.

15. Zubereitung nach einem der vorhergehenden Ansprüche, **dadurch gekennzeichnet, dass** die Zubereitung Calciumstärkeoctenylsuccinat in einer Konzentration von 0,5 bis 10 Gewichts-%, bezogen auf das Gesamtgewicht der Zubereitung, enthält.

## Claims

1. Cosmetic preparation comprising
a) calcium chloride and/or calcium carbonate at a total concentration of 0.01 to 3% by weight, based on the total weight of the preparation,
b) glycerin,
c) calcium starch octenylsuccinate (INCI Calcium Starch Octenylsuccinate).

2. Preparation according to Claim 1, **characterized in that** the calcium salt used is calcium chloride.

3. Preparation according to either of the preceding claims, **characterized in that** the preparation comprises glycerin at a concentration of 1 to 30% by weight, based on the total weight of the preparation.

4. Preparation according to any of the preceding claims, **characterized in that** the preparation is in the form of an emulsion.

5. Preparation according to any of the preceding claims, **characterized in that** the preparation is in the form of an O/W emulsion.

6. Preparation according to any of the preceding claims, **characterized in that** the preparation comprises one or more emulsifiers selected from the group of compounds comprising polyglyceryl-10 stearate, glyceryl stearate citrate, glyceryl stearate, glyceryl stearate (self-emulsifying), polyglyceryl-3 methylglucose distearate, sodium cetearyl sulfate, potassium cetyl phosphate, sodium stearoyl glutamate, triceteareth-4 phosphate, stearic acid, stearate salts, cetearyl sulfosuccinate.

7. Preparation according to any of the preceding claims, **characterized in that** the preparation comprises one or more active ingredients selected from the group of compounds comprising glycyrrhetic acid, urea, arctiin, alpha-lipoic acid, folic acid, phytoene, D-biotin, coenzyme Q10, hyaluronic acid, alpha-glucosylrutin, carnitine, carnosine, caffeine, natural and/or synthetic isoflavonoids, glyceryl glucoside, creatine, creatinine, taurine, β-alanine and/or licochalcone A, panthenol, tocopherol, tocopherol acetate, vitamin C, vitamin C derivatives, Glycyrrhiza inflata root extract, magnolia extract, aloe vera, urea, Arctium lappa fruit extract.

8. Preparation according to any of the preceding claims, **characterized in that** the preparation comprises propylene glycol, butylene glycol, 2-methylpropane-1,3-diol, ethylhexylglycerin, pentane-1,2-diol, hexane-1,2-diol, octane-1,2-diol and/or decane-1,2-diol.

9. Preparation according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; 4-(tert-butyl)-4'-methoxydibenzoylmethane; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; 4-(tert-butyl)-4'-methoxydibenzoylmethane; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; titanium dioxide; zinc oxide.

10. Preparation according to any of the preceding claims, **characterized in that** the preparation is free from 2-ethylhexyl 2-cyano-3,3-diphenylacrylate; 2-ethylhexyl 4-methoxycinnamate; isoamyl 4-methoxycinnamate; 2-hydroxy-4-methoxybenzophenone; 2-hydroxy-4-methoxy-4'-methylbenzophenone; 3-(4-methylbenzylidene)camphor and 3-benzylidenecamphor, propylparaben and butylparaben, isothiazolinones and 3-iodopropargyl N-butylcarbamate (IPBC).

11. Preparation according to any of the preceding claims, **characterized in that** the preparation comprises one or more perfumes selected from the group of compounds comprising limonene, citral, linalool, alpha-isomethyl ionone, geraniol, citronellol, 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic diesters, alpha-amyl cinnamaldehyde, alpha-methyl ionone, amyl C butylphenyl methylpropional cinnamal, amyl salicylate, amyl cinnamyl alcohol, anise alcohol, benzoin, benzyl alcohol, benzyl benzoate, benzyl cinnamate, benzyl salicylate, bergamot oil, bitter orange oil, butylphenyl methylpropional, cardamom oil, cedrol, cinnamal, cinnamyl alcohol, citronellyl methylcrotonate, lemon oil, coumarin, diethyl succinate, ethyl linalool, eugenol, Evernia furfuracea extract, Evernia prunastri extract, ethylene brassylate, farnesol, guaiac wood oil, hexyl cinnamal, hexyl salicylate, hydroxycitronellal, lavender oil, limonene oil, linalyl acetate, mandarin oil, menthyl PCA, methylheptenone, nutmeg oil, rosemary oil, sweet orange oil, terpineol, tonka bean oil, triethyl citrate, vanillin.

12. Preparation according to any of the preceding claims, **characterized in that** the preparation is free from polyethylene glycol, polyethylene glycol ethers and polyethylene glycol esters (so-called PEG derivatives).

13. Preparation according to any of the preceding claims, **characterized in that** the preparation comprises myristyl alcohol, stearyl alcohol and/or cetyl alcohol.

14. Preparation according to any of the preceding claims, **characterized in that** the preparation comprises phenoxyethanol.

15. Preparation according to any of the preceding claims, **characterized in that** the preparation comprises calcium starch octenylsuccinate at a concentration of 0.5 to 10% by weight, based on the total weight of the preparation.

## Revendications

1. Préparation cosmétique contenant
a) du chlorure de calcium et/ou du carbonate de calcium à une concentration totale de 0,01 à 3 % en poids, par rapport au poids total de la préparation,
b) du glycérol,
c) du succinate octénylique d'amidon calcique (INCl Calcium starch Octenylsuccinate).

2. Préparation selon la revendication 1, **caractérisée en ce que** du chlorure de calcium est utilisé en tant que sel de calcium.

3. Préparation selon l'une quelconque des revendications précédentes, **caractérisée en ce que** la préparation contient du glycérol à une concentration de 1 à 30 % en poids, par rapport au poids total de la préparation.

4. Préparation selon l'une quelconque des revendications précédentes, **caractérisée en ce que** la préparation se trouve sous forme d'une émulsion.

5. Préparation selon l'une quelconque des revendications précédentes, **caractérisée en ce que** la préparation se trouve sous forme d'une émulsion H/E.

6. Préparation selon l'une quelconque des revendications précédentes, **caractérisée en ce que** la préparation contient un ou plusieurs émulsifiants choisis dans le groupe des composés stéarate de polyglycéryle-10, stéarate-citrate de glycéryle, stéarate de glycéryle, stéarate de glycéryle (auto-émulsifiant), distéarate de polyglycéryl-3-méthylglucose, cétéarylsulfate de sodium, cétyl-phosphate de potassium, stéaroylglutamate de sodium, tricétéaréth-4-phosphate, acide stéarique, sels stéarates, cétéarylsulfosuccinates.

7. Préparation selon l'une quelconque des revendications précédentes, **caractérisée en ce que** la préparation contient une ou plusieurs substances actives choisies dans le groupe des composés acide glycyrrhétinique, urée, arctiine, acide alpha-lipoïque, acide folique, phytoène, D-biotine, coenzyme Q10, acide hyaluronique, alpha-glycosylrutine, carnitine, carnosine, caféine, isoflavonoïdes naturels et/ou synthétiques, glycérylglucoside, créatine, créatinine, taurine, β-alanine et/ou licochalcone A, panthénol, tocophérol, acétate de tocophérol, vitamine C, dérivés de vitamine C, extrait de racine de Glycyrrhiza inflata, extrait de magnolia, Aloe vera, urée, extrait de fruit d'Arctium lappa.

8. Préparation selon l'une quelconque des revendications précédentes, **caractérisée en ce que** la préparation contient du propylèneglycol, du butylène-glycol, du 2-méthylpropane-1,3-diol, de l'éthylhexylglycérol, du 1,2-pentanediol, du 1,2-hexanediol, du 1,2-octanediol et/ou du 1,2-décanediol.

9. Préparation selon l'une quelconque des revendications précédentes, **caractérisée en ce que** la préparation contient un ou plusieurs filtres UV, choisis dans le groupe des composés acide 2-phénylbenzimidazole-5-sulfonique et/ou ses sels ; sels d'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels d'acide 4-(2-oxo-3-bornylidèneméthyl)benzènesulfonique ; sels d'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; salicylate d'éthylhexyle ; acide téréphtalidènedicamphosulfonique ; 2-cyano-3,3-diphénylacrylate de 2-éthylhexyle ; 4-(diméthylamino)-benzoate de 2-éthylhexyle ; 4-(diméthylamino)benzoate d'amyle ; 4-méthoxybenzalmalonate de di(2-éthylhexyle) ; 4-méthoxycinnamate de 2-éthylhexyle ; 4-méthoxycinnamate d'isoamyle ; 2-hydroxy-4-méthoxybenzophénone, 2-hydroxy-4-méthoxy-4'-méthylbenzophénone ; 2,2'-dihydroxy-4-méthoxybenzophénone ; 4-(tert.-butyl)-4'-méthoxydibenzoylméthane ; salicylate d'homomenthyle ; 2-hydroxybenzoate de 2-éthylhexyle ; diméthicodiéthylbenzalmalonate ; copolymère 3-(4-(2,2-bis-éthoxy-carbonylvinyl)-phénoxy)propényl)-méthoxysiloxane / diméthylsiloxane ; 4-(tert.-butyl)-4'-méthoxydibenzoylméthane ; 2-(4'-diéthylamino-2'-hydroxybenzoyl)benzoate d'hexyle ; dioctylbutylamidotriazone (INCI : Diethylhexyl-Butamidotriazone) ; 2,4-bis-[5-1(diméthylpropyl)benzoxazol-2-yl-(4-phenyl)-imino]-6-(2-éthylhexyl)-imino-1,3,5-triazine portant le (n° CAS 288254-16-0) ; 2,4-bis-{[4-(2-éthylhexyloxy)-2-hydroxy]-phenyl}-6-(4-méthoxyphényl)-1,3,5-triazine (INCI : Bis-Ethylhexyloxyphenol Methoxyphenyl Triazin) ; 4,4',4"-(1,3,5-triazine-2,4,6-triyltriimino)-tris-benzoate de tris(2-éthylhexyle) (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; dioxyde de titane ; oxyde de zinc.

10. Préparation selon l'une quelconque des revendications précédentes, **caractérisée en ce que** la préparation est exempte de 2-cyano-3,3-diphénylacrylate de 2-éthylhexyle ; 4-méthoxycinnamate de 2-éthylhexyle ; 4-méthoxycinnamate d'isoamyle ; 2-hydroxy-4-méthoxybenzophénone ; 2-hydroxy-4-méthoxy-4'-méthylbenzophénone ; 3-(4-méthylbenzylidène)camphre et 3-benzylidènecamphre, propyl- et butyl-parabènes, isothiazolinones et N-butylcarbamate de 3-iodopropargyle (IPBC).

11. Préparation selon l'une quelconque des revendications précédentes, **caractérisée en ce que** la préparation contient un ou plusieurs parfums choisis dans le groupe des composés limonène, citral, linalool, alpha-isométhylionone, géraniol, citronellol, 2-isobutyl-4-hydroxy-4-méthyltétrahydropyrane, acétate de 2-tert-pentylcyclohexyle, 3-méthyl-5-phényl-1-pentanol, 7-acétyl-1,1,3,4,4,6-hexaméthyltétraline, diester d'acide adipique, alpha-amylcinnamaldéhyde, alpha-méthylionone, amyl C butylphenylméthylpropionalcinnamal, salicylate d'amyle, alcool amylcinnamylique, alcool anisique, benzoïne, alcool benzylique, benzoate de benzyle, cinnamate de benzyle, salicylate de benzyle, huile de bergamote, huile d'orange amère, butylphenylméthylpropioal, huile de cardamome, cédrol, cinnamal, alcool cinnamylique, crotonate de citronellylméthyle, huile de citron, coumarine, succinate de diéthyle, éthyllinalool, eugénol, extrait d'Evernia furfuracea, extrait d'Evernia prunastri, brassylate d'éthylène, farnésol, huile de bois de guaïac, hexylcinnamal, salicylate d'hexyle, hydroxycitronellal, huile de lavande, huile de lémonène, acétate de linayle, huile de mandarine, menthyl PCA, méthylhepténone, huile de noix de muscade, huile de romarin, huile d'orange douce, terpinéol, huile de fèves tonka, citrate de triéthyle, vanilline.

12. Préparation selon l'une quelconque des revendications précédentes, **caractérisée en ce que** la préparation est exempte de polyéthylèneglycol, éthers de polyéthylèneglycol et esters de polyéthylèneglycol (les dénommés dérivés de PEG).

13. Préparation selon l'une quelconque des revendications précédentes, **caractérisée en ce que** la préparation contient de l'alcool myristylique, de l'alcool stéarylique et/ou de l'alcool cétylique.

14. Préparation selon l'une quelconque des revendications précédentes, **caractérisée en ce que** la préparation contient du phénoxyéthanol.

15. Préparation selon l'une quelconque des revendications précédentes, **caractérisée en ce que** la préparation contient du succinate octénylique d'amidon calcique à une concentration de 0,5 à 10 % en poids, par rapport au poids total de la préparation.
